# EUROPEAN PATENT SPECIFICATION

(11) **EP 1 364 642 B1**
(45) Date of publication and mention of the grant of the patent: **09.08.2006**
(21) Application number: 03253186.5
(22) Date of filing: 21.05.2003
(51) Int. Cl.: A61K 9/107, A61K 9/127, A61K 31/05

(54) **Composition for delivery of dithranol**
Zubereitung zur Verabreichung von Dithranol
Composition pour l'administration du dithranol

(30) Priority: 23.05.2002 US 382997 P; 21.02.2003 US 371295
(43) Date of publication of application: 26.11.2003
(73) Proprietor: USV Limited, Mumbai 400088, Maharashtra (IN)
(72) Inventor: Gidwani, Suresh Kumar, Mumbai-400076, Maharashtra (IN); Singnurkar, Purushottam Sharshikant, Yawal Distr. Jalgaon, Maharashtra-425323 (IN)
(74) Representative: Beresford, Keith Denis Lewis

(56) References cited:
- WO-A-95/13052
- US-A- 5 260 065
- US-A- 5 358 716
- GEHRING W ET AL: "Enhancement of the penetration of dithranol and increase of effect of dithranol on the skin by liposomes." ARZNEIMITTEL-FORSCHUNG. GERMANY JUL 1992, vol. 42, no. 7, July 1992 (1992-07), pages 983-985, XP008020889 ISSN: 0004-4172

## Description

### BACKGROUND OF THE INVENTION

Dithranol (1,8,9- trihydroxyanthracene) is used in the topical treatment of psoriasis, eczemas, dermatophytoses, alopecia areata and other dermatological diseases. It suffers however from some serious disadvantages.
■ It is highly irritant to skin not affected by psoriasis and must therefore be applied with considerable care and often limits the time of exposure of such preparations to the skin. Currently in clinical practice the preparations containing dithranol are to be applied on the skin for short periods and are to be removed after 30 minutes of application otherwise it becomes very difficult to tolerate the skin irritation.
■ It is easily oxidized to brown or black products and has a powerful staining effect on clothing and normal skin. Commercially in European countries it is known that dithranol is mixed with ointment base and compounded by the pharmacist in the pharmacy and given to the patient for immediate use. Also British Pharmacopoeia 2001, volume II, page 2035 states that Dithranol Cream should be stored at a temperature not exceeding 25°C.
■ It is very difficult to deliver dithranol in soluble form in the concentration ranging above 0.15% and it is the soluble form of the dithranol which is more effective than dispersed or suspended dithranol.

There is a need to have dithranol compositions which will be free of above undesirable effects, and which can be applied to the skin for a prolonged period of time to achieve better therapeutic effect in the treatment of psoriasis. Also such preparations should be stable on prolonged storage.

There have been a number of references in the literature to compositions containing dithranol. Such as
■ Ointments (petroleum base).
■ Creams with either o/w or w/o emulsion.
■ Liposomes & Niosomes formulations (Commercially not available).
■ Solid lipid nanoparticles (Commercially not available).

However they have their own limitations one of the other with respect to stability, efficiency, irritation potential & staining caused by dithranol. One of the major limitations is the low level of dithranol in soluble form which often requires dispersion of dithranol in powder form to achieve dithranol concentration above 0.75% in the product.

Laugier; Jean Pierre et al. in US Patent No. 5358716 described oil in water emulsion containing dithranol and nonionic vesicles prepared for nonionic amphilic lipids, in order to prevent the oxidation of the dithranol. Vesicles are dispersed in the aqueous phase of the emulsion. However, dithranol is not encapsulated in the vesicles and the o/w emulsion compositions with dithranol content greater than 0.15% are present as micronised dithranol in dispersed form and not in solution form. It is not possible to incorporate dithranol in solution form above 0.15% concentration in such preparations due to limitation of dithranol solubility in oil and lipid phase.

Shyamal, C et al., Journal of Pharmaceutical Science Vol. 85, No. 10, October 1996, 1082-1084; described the topical delivery of Erythromycin from Liposomal emulsion. A nonionic liposomal formulation containing glyceryl dilaurate, cholesterol, polyoxyethylene -10-stearyl ether at weight ratio of 57:15:28 was prepared by melting the lipid phase containing Erythromycin base at 60° C followed by hydration with buffer solution. This liposomal dispersion was mixed with mineral oil or isopropyl palmitate and sonicated to yield mixture of emulsion with liposomes. They showed maximum stability for 2 weeks for such formulations. It is known in the art that any active ingredient/drug present in solution form when applied topically, diffuses more rapidly through the skin as compared to dispersed solid particulate drug. It can very well be demonstrated by universal Fick's law of diffusion. It was an object of the invention to overcome the above-mentioned limitations and to formulate efficient and stable delivery system containing dithranol alone or in combination with salicylic acid and coal tar extract for effective and rapid control of psoriasis.

### SUMMARY OF INVENTION

The present invention relates to a mixed vesicular system for the topical delivery of dithranol alone or in combination with salicylic acid which mimics niosomes and liposomes. This system, if desired, can also contain vacuum dried coal tar extract. The mixed vesicular system is composed of a nanoemulsion with bilayer nanovesicles, wherein dithranol and, if present, salicylic acid are entrapped in the vesicles. This is achieved through the use in this system of a nonionic oily liquid lipid material capable of solubilizing dithranol alone or dithranol in combination with salicylic acid. The mixed vesicular system preferably has particle size of no greater than 450 nm and can be stored at room temperature for extended periods of time . Furthermore the composition of this invention does not produce irritation or staining and is very effective for the treatment of psoriasis.

It is known that any active ingredient/drug present in solution form diffuses rapidly through the skin as compared to solid dispersed particulate drug. The composition of this invention provides topical pharmaceutical compositions containing dithranol in soluble form even at higher drug concentrations so as to make it more effective. In this manner, the active ingredients can penetrate into and through the skin so that the active ingredients are provided continuously and in sufficient quantity at the site of action. The topical compositions of this invention can be used for the topical treatment of psoriasis, eczemas, dermatophytoses, alopecia areata and other dermatological diseases In addition, this invention provides a topical composition which is stable at 37°C to 45°C for more than six months thus making it readily dispensable and able to be stored at room temperature in tropical countries.

### DETAILED DESCRIPTION

In a first aspect, the invention provides a composition containing solubilized dithranol, said composition comprising a mixture of bilayer vesicles in an oil and water nanoemulsion, wherein an oily lipid phase of said bilayer vesicles and of said nanoemulsion is comprised of a non-ionic oily liquid lipid material that solubilizes dithranol;
characterised in that:
said non-ionic oily liquid lipid material is comprised of α-tocopherol acetate alone or in combination with polyoxypropylene-15-stearyl ether,
and in that:
said bilayer vesicles are formed *in situ* in a high pressure homogenization step.

In a second aspect, the invention provides a composition containing solubilized dithranol, said composition comprising a mixture of bilayer vesicles in an oil and water nanoemulsion, wherein an oily lipid phase of said bilayer vesicles and of said nanoemulsion is comprised of a non-ionic oily liquid lipid material that solubilizes dithranol, the process comprising:
a) providing both a solution of dithranol dissolved in a non-ionic oily liquid lipid material and an aqueous medium;
b) mixing said oily solution with said aqueous medium to form a mixture;
c) homogenizing said mixture to form a homogeneous emulsion containing an oily phase and an aqueous phase, and
d) subjecting said homogeneous mixture to high pressure homogenization at pressures from 138 MPa to 207 MPa (20,000 psi to 30,000 psi);
characterised in that:
said non-ionic oily liquid lipid material is comprised of α-tocopherol acetate alone or in combination with polyoxypropylene-15-stearyl ether,
and in that:
said bilayer vesicles are formed *in situ* in said high pressure homogenization step (d).

Preferred embodiments are defined in the dependent claims. For example, the composition can contain either dithranol or a mixture of dithranol with salicylic acid solubilized in the lipid phase. In accordance with this invention either dithranol alone or with salicylic acid can form the active ingredient in the topical treatment of dermatological diseases such as psoriasis, etc. In accordance with this invention, the dithranol present in the system in concentrations as high as 1.0% by weight of said composition can be completely solubilized in this composition. In general, these compositions contain dithranol in an amount ranging from about 0.1 % to about 1% by weight based upon the weight of the composition. If it is desired to incorporate salicylic acid in the composition, the salicylic acid is present in combination with dithranol in an amount of from about 0.1% to about 3% by weight based upon the weight of the composition and preferably from about 0.5% to about 1.5% by weight based upon the weight of said composition. If salicylic acid is present in combination with dithranol, this combination is entrapped in the lipid phase of the bilayer vesicles within the nanoemulsion.

Solublization is important since dithranol is insoluble in water and sparingly soluble in hydrocarbon bases, vegetable oils and esters of fatty acids; limiting its concentration below 0.1% in soluble form. It has been very difficult to formulate preparations containing dithranol above 0.1% w/w concentration in soluble form. Surprisingly, it has been found that dithranol and combinations of dithranol and salicylic acid can be solubilized in non-ionic liquid lipid materials such as tocopherol acetate (Vitamin E acetate) which can be used as a vehicle to keep the dithranol in solution in stable form even at high concentrations.

In accordance with the present invention it is discovered that by using α-tocopherol acetate, a non-ionic oily liquid lipid material which acts as a solubilizer for dithranol, the dithranol and combinations of dithranol and salicylic acid will be kept in solution entrapped in the vesicles in this oily lipid material which forms the oily phase in both the nanoemulsion and the bilayer vesicles. The non-ionic oily liquid lipid material may be a mixture of α-tocopherol acetate and Arlamol E® (Polyoxypropylene-15-stearyl ether), with α-tocopherol acetate being preferred. In formulating the system of this invention, the non-ionic oily liquid lipid material can be generally present in an amount of from about 3% to 40% by weight based upon the weight of the composition, with amounts of from about 5% to 30% by weight based upon the weight of the composition being specially preferred.

The system of this invention achieves enhanced encapsulation of dithranol in solubilized state. Furthermore the composition of the invention surprisingly has been found to be free of undesirable side effects, and provides excellent therapeutic effects in psoriasis, eczemas, dermatophytoses, alopecia areata and other skin disorders when it is administered topically. In clinical tests it has been found that the system of this invention due to the presence of micro-emulsion with mixed vesicles provides effective delivery of dithranol. These advantages are in some part due to -
■ Presence of nanoemulsion instead of emulsion.
■ Presence of combination of micro-emulsion with mixed vesicles.
■ Dithranol entrapment in soluble form.
■ Preparation mimics liposomal and niosomal delivery system.
It is known that any active ingredient/drug present in solution form diffuses rapidly through the skin as compared to solid dispersed particulate drug. It is an object of the invention to make pharmaceutical compositions containing dithranol in soluble form even at higher drug concentrations so as to make it more effective, whereby the active ingredient can penetrate into and through the skin so that the active ingredients are provided continuously and in sufficient quantity at the site of action.

In accordance with this invention, the composition for topical administration is in the form of a homogeneous mixture of an oil and water nanoemulsion that constitutes an oil lipid phase and an aqueous phase. It can be either an oil in water or water in oil emulsion in which spherical hollow bilayer vesicles like liposomes are present in the single system wherein the dithranol or the dithranol in combination with salicylic acid is present in both the oil globules of the emulsion and in the oil or lipid phase of the bilayer vesicles in a soluble state. Generally, it is preferred that the composition of this invention be a homogeneous mixture of an oil in water nanoemulsion, with the oil lipid phase which is emulsified in the aqueous phase being present in an amount of from 8% to 50% by weight of the composition, preferably from about 5% to about 40% by weight of the composition.

The above mixed vesicular system of this invention which is present as a liquid, if desired, can be thickened to form a gel or cream. This can be done by adding a gelling agent which is a thickening agent or viscosity modifying agent to the liquid system.
Any conventional gelling agent can be utilized to convert the liquid emulsion of this invention into a cream or gel. Among the preferred gelling agents are included xanthan gum, ethylene oxide, carbopol, hydroxypropyl, methyl or cellulose. The amount of gelling agent to be added to the composition depends upon the ultimate viscosity of the gel or cream that is desired.

In accordance with one embodiment of this invention when salicylic acid is used in combination with dithranol the salicylic acid is also retained in soluble form along with dithranol in the mixed vesicular system mimicking niosomes and liposomes. It is also known in the art that pharmaceutical compositions containing dithranol are more effective in combination with salicylic acid and alcoholic extract of coal tar for the treatment of psoriasis. However such preparations are reported to have some percentage of alcohol content which may intensify the irritation potential to the skin. In addition, if it is desired to further reduce any possible additional irritating effect of alcohol on the skin of the topical composition of this invention one can vacuum dry the alcoholic extract of coal tar and add it to the composition of this invention. This vacuum dried coal tar extract is in the form of viscous slurry. Generally in accordance with a preferred embodiment of this invention the coal tar extract is added in an amount of from about 0.1% to about 6.0 %, by weight based on the weight of the composition with amounts of from about 0.25% to about 2.0% by weight being especially preferred.

It is known in the art that dithranol is very sensitive to oxidative degradation and a variety of known antioxidants such as butylated hydroxyanisole, butylated hydroxytoulene, ascorbic acid, citric acid, oxalic acid, tartaric acid, succinic acid etc.can be present in the preferred compostion of this invention In the present invention, it is preferred to use antioxidants such as BHA and BHT in combination with ascorbic acid to prevent oxidative degradation of dithranol. In one embodiment of the present invention dithranol alone or in combination with salicylic acid is dissolved in a mixture of tocopherol acetate and Arlamol E® (Polyoxypropylene-15-stearyl ether) containing nonionic surfactant and cholesterol. Thus the oily phase formed was homogenized with aqueous phase

It is known that any active ingredient/drug present in solution form diffuses rapidly through the skin as compared to solid dispersed particulate drug. In accordance with this invention topical pharmaceutical compositions are prepared containing dithranol in soluble form even at higher drug concentrations than previously achievable so as to make it more effective. In this manner the active ingredients can penetrate into and through the skin so that they are provided continuously and in sufficient quantity at the site of action.

The oil phase may contain lecithin (Phosphotidyl choline) or other synthetic, semisynthetic lecithins alone or in combination such as hydrogenated phosphatidyl choline, Phosphatidylethanolamine, Dipalmatoyl phosphatidyl choline (DPPC), Dipalmatoyl phosphatidylethanolamine (DPPE) Distearoyl phosphatidylcholine (DSPC), Distearoyl phosphatidylethanolamine (DSPE), Dioleylphosphatidylcholine (DOPC), Dioleylphosphatidylethanolamine (DOPE), Phosphatidic acid (PA), Phosphatidylserine (PS), Phosphatidylglycerol (PG) and their hydrogenated analogs.

The preferred carrier for dithranol or dithranol with salicylic acid in accordance with the present invention is tocopherol acetate or mixture of tocopherol acetate. The concentration of tocopherol acetate in the system used for dissolution of dithranol may range from about 5% to about 40% by weight based on the weight of the total composition. The preferred concentration range, however, is from about 3% to about 30%. The concentration of Arlamol E® (Polyoxypropylene-15-stearyl ether) may range from about 1% to about 10% by volume, based upon the volume of the total composition. The preferred concentration range, however, is from about 3% to about 7%.

In the present invention non-ionic surfactant refers to fatty acid esters and ethers of triglycerides, diglycerides or monoglycerides having HLB value in the range of 4 to 16 preferably between 8 to 14. Examples of non-ionic surfactant include but are not limited to polyoxyl 60 hydrogenated castor oil (Cremophore® RH-60), polyoxyl 35 hydrogenated castor oil (Cremophore® EL), polyoxyl 40 hydrogenated castor oil (Cremophore® RH-40), sorbitan monooleate (Span-80), polyoxyethylene 20 sorbitan monooleate (Tween-80), Span 20, polyglyceryl-3-oleate (Plurol Olique®) PEG-32 glyceryl stearate (Gelucire53/10).

The concentration of non-ionic surfactant may range from about 0.5% to about 5% by volume based upon the volume of the total composition. The preferred concentration range however is from about 0.75% to about 3% by volume.

The concentration of cholesterol may range from about 0.1 to about 2% by volume of the composition, preferably from about 0.5 to about 1.5%. The lecithin may be present in the mixed vesicular system and the concentration may range from 0.2 to 10% by volume of the total composition, preferably from about 1% to about 6%.

In accordance with embodiments of this invention other commonly used additives known in the art such as antioxidants, chelating agent may be present. Antioxidants which can be included in accordance with an embodiment of this invention are combinations of butylated hydroxyanisole, butylated hydroxytoulene and ascorbic acid chelating agents including Disodium EDTA. The concentration of antioxidants and chelating agent may range from about 0.01% to about 0.5 % by volume of the total composition, preferably about 0.1% to about 0.5% by volume.

The particle size of the mixed vesicular system obtained by present invention is preferably no greater than 450 nm. Accordingly, in another embodiment of the present invention, the mixed vesicular system is preferably gelled. Among the gelling agents which can be used are cellulose derivatives such as hydroxypropyl methyl cellulose, carbopol, xanthan gum and other hydrophilic polymer such as polyethylene oxide. Depending upon the desired viscosity, the gelling agent may be generally added in amounts from about 0.1% to about 5% by weight based upon the weight of the composition, preferably 0.5 to about 3% by weight. Concentration of dithranol in the mixed vesicular system according to an embodiment of the present invention may range from 0.01 to about 1.0% by weight in soluble form.

Concentration of salicylic acid in the mixed vesicular system according to an embodiment of the present invention may range from about 0.1% to about 3.0% in soluble form, preferably from about 0.5% to 1.5% by weight. The vacuum dried coal tar extract according to the present invention can be prepared by first dispersing and soaking the coal tar viscous mass in an alcoholic solution of polyoxyethylene 20 sorbitan monolaurate (Tween-80) for 7 days. The extract is then filtered and alcohol is removed from the filtrate by vacuum evaporation at 35°C temperature in rotary vacuum evaporator to get vacuum dried coal tar extract. Concentration of vacuum dried coal tar extract in the mixed vesicular system according to the present invention may range from about 0.01% to about 6.0 % by weight based upon the weight of the composition, preferably from about 0.25% to about 2% by weight.

This invention is directed to the process for preparing an oil and water nanoemulsion with an oil and water phase wherein the nanoemulsion has dispersed therein a plurality of bilayer vesicles retaining dithranol alone or in combination with salicylic acid solubilized in the oily layers of the vesicles. In accordance with this invention, the nanoemulsion is carried out providing a solution of dithranol dissolved in the non-ionic oily liquid lipid material as well as a separate aqueous medium which may contain such excipients as stabilizers, anti-oxidants, etc. dissolved therein. The aqueous medium and the oily solution are mixed together and then homogenized to form a homogeneous emulsion which contains the oily phase and the aqueous phase. Any conventional homogenizer may be used to homogenize this emulsion mixture. This emulsion is next subjected to high pressure homogenization at pressures from about 138 MPa to about 207 MPa (about 20,000 psi to about 30,000 psi). In the final step, the homogeneous mixture after being subjected to high pressure homogenization is passed through a 0.45 µm membrane. In this manner, the oil and water nanoemulsion is produced. The oily non-ionic lipid medium α-tocopherol acetate, prior to mixing with the aqueous phase, contains dithranol or dithranol in combination with salicylic acid solubilized in said lipid phase which optionally contains polyoxypropylene-15-stearyl ether.

This homogenized mixture is then passed through high pressure homogenizer at 138 to 207 MPa (20,000 to 30,000 psi) pressure followed by passing through 0.45 µm membrane. The resulting system was found to be a mixed vesicular system comprising nanoemulsion with vesicles formed by combination of tocopherol acetate, nonionic surfactant, cholesterol and lecithin, encapsulating the dithranol alone or in combination with salicylic acid in soluble form. In the present invention, the oil phase composition is such that after emulsification and high pressure homogenization it forms an oil in water emulsion along with *in situ* formation of bilayer vesicles composed of the non-ionic oily liquid lipid material α-tocopherol acetate optionally with polyoxypropylene-15-stearyl ether. In accordance with the method of this invention, the bilayer vesicles are formed *in situ* after the high pressure homogenization step and are not added separately to the system.

The composition according to the present invention having 0.5% w/w dithranol, 1.15% w/w salicylic acid and 0.58 %,w/w vacuum dried coal tar extract were tested clinically on 12 patients with skin psoriasis and found to be equally as effective as compared to conventional dithranol ointment containing 1.1% w/w dithranol, 1.15% w/w salicylic acid & 5.3% w/w alcoholic extract of coal tar solution.

### Examples

The following examples set forth herein below illustrate the invention:

### Example 1

| Part A: | | |
|---|---|---|
| Dithranol | -- | 0.5% w/w |
| Salicylic acid | -- | 1.15 w/w |
| α-Tocopherol acetate | -- | 15% w/w |
| Polyoxypropylene-15-Stearyl ether (Arlamol®-E) | -- | 5% w/w |
| Oleic acid | -- | 0.5% w/w |
| Butylated hydroxy anisole | -- | 0.1% w/w |
| Butylated hydroxy toluene | -- | 0.1% w/w |
| Cholesterol | -- | 0.5% w/w |
| Polyoxyl 40 hydrogenated castor oil | -- | 1.0% w/w |
| Egg lecithin (Lipoid® E-8o S) | -- | 3.0% w/w |
| Ethanol | -- | 0.75% w/w |
| | | |

| Part B: | | |
|---|---|---|
| Glycerin | -- | 5.0% w/w |
| Disodium EDTA | -- | 0.1% w/w |
| Ascorbic acid | -- | 0.5% w/w |
| Demineralised water | -- | 51.0% w/w |
| | | |

| Part C: | | |
|---|---|---|
| Xanthan gum | -- | 2.0% w/w |
| Methyl paraben | -- | 0.1% w/w |
| Propyl paraben | -- | 0.02% w/w |
| Sodium hydroxide | -- | 0.06% w/w |
| Demineralised water | -- | 13.0% w/w |
| | | |

| Part D: | | |
|---|---|---|
| Coal tar extract (Vacuum drier) | -- | 0.58% w/w |

■ Heat Part A to 50° C under nitrogen gas flushing and mix to get homogenous solution. Then cool to room temperature.
■ Mix Part B with homogenizer.
■ Add Part A into Part B with homogenization. Then transfer the mixture to high pressure homogenizer (APV Gaulin LAB 40) and homogenize it for 10-12 passes at 207 MPa (30,000 psi) pressure. The resulting dispersion is passed through 0.45 µm membrane to obtain the mixed vesicular system with average particle size less than 450 nm.
■ Part C, xanthan gum gel is prepared separately by hydrating xanthan gum in demineralised water containing parabens at 80-90° C temperature and allow to cool and soak at room temperature. Then add above homogenized mixed vesicular system and mix to get the gel consistency. Finally add coal tar exact slurry (vacuum dried) and mix thoroughly to get the final composition.

### Accelerated Stability Study:

The mixed vesicular composition of Example 1 was filled in collapsible tubes and kept at accelerated temperature conditions, 25°C, 37°C and 45°C for 6 months. Samples were periodically analysed for Dithranol and Salicyclic acid content by HPLC analysis. The results are tabulated in the following table.

| Period | Storage Temperature | Dithranol content % by weight | Salicylic acid content % by weight |
|---|---|---|---|
| Initial | ------ | 0.518 | 1.159 |
| 1 Month | 25°C | 0.521 | 1.159 |
| | 37°C | 0.518 | 1.158 |
| | 45°C | 0.517 | 1.156 |
| 3 Month | 25°C | 0.511 | 1.156 |
| | 37°C | 0.509 | 1.153 |
| | 45°C | 0.507 | 1.1516 |
| 6 Month | 25°C | 0.508 | 1.155 |
| | 37°C | 0.501 | 1.1514 |
| | 45°C | 0.494 | 1.148 |

Conclusion The mixed vesicular composition is stable over 6 month even at 37°C and 45°C temperature storage as evidenced by it's dithanol content and salicylic acid content not reducing drastically as compared to the initial value.

### Example 2

| Part A: | | |
|---|---|---|
| Dithranol | -- | 0.5% w/w |
| α-Tocopherol acetate | -- | 15% w/w |
| Polyoxypropylene-15- Stearyl ether (Arlamol®-E) | -- | 5% w/w |
| Oleic acid | -- | 0.5% w/w |
| Butylated hydroxy anisole | -- | 0.1% w/w |
| Butylated hydroxy toluene | -- | 0.1% w/w |
| Cholesterol | -- | 0.25% w/w |
| Polyoxyl 40 hydrogenated castor oil | -- | 1.0% w/w |
| Egg lecithin (Lipoid® E-8o S) | -- | 3.0% w/w |
| Ethanol | -- | 0.75% w/w |
| | | |

| Part B: | | |
|---|---|---|
| Glycerin | -- | 5.0% w/w |
| Disodium EDTA | -- | 0.1% w/w |
| Ascorbic acid | -- | 0.5% w/w |
| Demineralised water | -- | 51.58% w/w |
| | | |

| Part C: | | |
|---|---|---|
| Xanthan gum | -- | 2.0% w/w |
| Methyl paraben | -- | 0.1% w/w |
| Propyl paraben | -- | 0.02% w/w |
| Sodium hydroxide | -- | 0.06% w/w |
| Demineralised water | -- | 13.0% w/w |

■ Heat Part A to 50° C under nitrogen gas flushing and mix to get homogenous solution. Then cool to room temperature.
■ Mix Part B with homogenizer.
■ Add Part A into Part B with homogenization. Then transfer the mixture to high pressure homogenizer (APV Gaulin LAB 40) and homogenize it for 10-12 passes at 207 MPa (30,000 psi) pressure. The resulting dispersion is passed through 0.45 µm membrane to obtain the mixed vesicular system with average particle size less than 450 nm.
■ Part C, xanthan gum gel is prepared separately by hydrating xanthan gum in demineralised water containing parabens at 80-90° C temperature and allow to cool and soak at room temperature. Then add above homogenized mixed vesicular system and mix to get the gel consistency.

### Example 3

[Therapeutic composition without Polyoxypropylne-15-stearyl ether
(Arlamol®-E)]

Therapeutic composition similar to that described in Example 1, except α-tocopherol acetate was increased from 15% to 20% by weight and Polyoxypropylne-15-stearyl ether (Arlamol®-E) was omitted.

### Example 4

Therapeutic composition similar to that described in Example 1, except 3.0% w/w Egg lecithin (Lipoid E 80 S) was replaced by Soya lecithin (Lipoid® S75).

### Example 5

Therapeutic composition similar to that described in Example 1, except 3.0% w/w Egg lecithin (Lipoid E 80 S) was replaced by Dipalmitoyl phosphatidylcholine (DPPC).

### Example 6

Therapeutic composition similar to that described in Example 1, except 1.0% w/w polyoxyl -40 hydrogenated castor oil was replaced by 1.0% w/w/ polysorbate 80.

### Example 7

Therapeutic composition similar to that described in Example 1, except 1.0% w/w polyoxyl-40 hydrogenated castor oil was replaced by 1.0% w/w span 20 (sarbitan monolaurate).

### Example 8

Therapeutic composition similar to that of Example 1, except 1.0% w/w polyoxyl-40-castor oil was replaced by 1.0% w/w polyglyceryl-3-oleate (Plurol Olique®)

### Example 9

Therapeutic composition similar to that of Example 1, except 0.5% w/w dithranol was substituted by 0.1% w/w dithranol and a-tocopherol acetate reduced from 15% w/w to 10% w/w.

### Example 10

Therapeutic composition similar to that of Example 1, except 0.5% w/w dithranol was substituted by 1.0% w/w dithranol.

### Example 11

### (Not forming the part of invention)

### Dithranol Ointment

| | | |
|---|---|---|
| Dithranol | -- | 1.15% w/w |
| Salicylic acid | -- | 1.15% w/w |
| Solution of coal tar (alcoholic) | -- | 5.3% w/w |
| Maize starch | -- | 12% w/w |
| White soft paraffin | -- | q.s. 100% |

Dithranol and maize starch were finely dispersed in melted white soft paraffin at 65-70° C temperature and cooled to room temperature with mixing. Salicylic acid was dissolved in coal tar solution and added to dithranol paraffin mixture & mixed thoroughly to get ointment consistency.

### Clinical Test:

Therapeutic compositions as described in Example 1, 2, and 11 were tested clinically and comparison is being made. Clinical test protocol was followed as described by Van Scott, Eugene J. et al. in U.S. Patent No. 4287214.

Skin involved in psoriasis is hyperplastic (thickened), erythematous (red or inflamed) and has thick adherent scales. The degree of thickening is such that lesions are elevated up to I mm above the surface of adjacent normal skin; erythema is usually an intense red; the thickened adherent scales cause the surface of involved skin to be marked by rough & uneven. These three attributes of thickness, colour & texture as described in US Patent No. 4287214 were quantified to allow comparative measurement of degree of improvement from topically applied therapeutic compositions - therapeutic composition of the present invention (Example 1 & Example 2) and conventional dithranol ointment (Example 11).

### Degree of Improvement

| | None | mid | Moderate | Substantial | Complete |
|---|---|---|---|---|---|
| | (o) | (1+) | (2+) | (3+) | (4+) |
| Thickness | Highly elevated | Detectable reduction | Readily apparent | Barely elevated | Normal thickness |
| Texture | Visibly rough | Palpably rough | Uneven but not rough | Slightly uneven | Visibly and palpably smooth |
| Colour | Intense Red | Red | Dark Pink | Light pink | Normal skin colour |

In order to ascertain whether the dithranol compositions of the present invention (Example 1 & Example 2) are therapeutically more efficacious than conventional Dithranol Ointment (Example 11) for topical treatment of psoriasis, a total of more than 12 patients having psoriasis were tested in this study.

The treatment areas in patients having psoriasis were localized lesions 8-20 cm in diameter. The therapeutic compositions were topically applied by the patient in an amount sufficient to cover the treatment. Applications were made two-three times daily and without occlusive dressing. Clinical evaluation of degree of improvement were made of weekly interval. The treatment was continued for 4 weeks unless clearing of disease occurred earlier & evaluation of degree improvement was made. The treatment results are summarized as follows

**TABLE 1: Effect on Psoriasis**

| | Dithranol concentration | Therapeutic | efficiency after |
|---|---|---|---|
| | | 2 weeks | 4 weeks |
| Example 1 | 0.5% w/w | + 3 | + 4 |
| Example 2 | 0.5% w/w | + 3 | + 4 |
| Example 11 | 1.1% w/w | + 2 | + 3 |

**TABLE 2: Staining and irritation effect of topically applied composition of skin**

| | Dithranol concentration | Irritation | Staining |
|---|---|---|---|
| Example 1 | 0.5%° w/w | No irritation | No staining |
| Example 2 | 0.5% w/w | No irritation | No staining |
| Example 11 | 1.1% w/w | High irritation observed, sometimes required for the removal from applied skin | Staining of cloth and skin observed |

### Conclusion

Therapeutic compositions of the present invention were found to be nonirritating, non-staining and therapeutically more effective even at low dithranol concentration (0.5% w/w) as compared to conventional Dithranol Ointment containing 1.1% w/w dithranol.

### DEFINITIONS

The terms "nanoemulsion" and "micro-emulsion" as used herein refer to submicron emulsions, which are emulsions having a dispersed phase that possesses a mean droplet diameter under 1 µm (See H.A. Lieberman, M.M. Rieger, G.S. Banker, "Pharmaceutical Dosage Forms: Disperse Systems," Volume 2, Second Edition, Revised and Expanded, Marcel Dekker, Inc., New York, 1996, page 49).

The term "vesicle" as used herein refers particularly to a bilayer vesicle which is a structure consisting of a sphere of lipid bilayers enclosing some of the aqueous phase. Several vesicles may be formed one inside the other in diminishing size, creating a multi-layer structure of concentric lipid bilayer spheres separated by layers of water or aqueous phase. They are similar to liposomes.

### References

| | | |
|---|---|---|
| U.S. Patent 3,981,996 | September 1976 | Leigh, et al. |
| U.S. Patent 5,904,932 | May 1976 | De Winger; Tom |
| U.S. Patent 4,954,345 | September 1990 | Muller; Joseph |
| U.S. Patent 5,061,486 | October 1991 | Whitefield; Martin |
| U.S. Patent 4,367,224 | January 1983 | Van Scott, et al. |
| U.S. Patent 5,894,019 | April 1999 | Hesse, et al. |
| U.S. Patent 4,895,727 | January 1990 | Allen; Larry M. |
| U.S. Patent 4,438,052 | March 1984 | Weder, et al. |
| U.S. Patent 6,328,988 | December 2001 | Uhrich; Kathryn E. |
| U.S. Patent 4,287,214 | September 1981 | Van Scott, et al. |
| U.S. Patent 4,203,969 | May 1980 | Yarrow, et al. |
| U.S. Patent 5,358,716 | October 1994 | Laugier, et al. |
| GB 2,157,173A | October 1985 | Josef Muller, et al. |

Shyamala, C.; Jayaraman, C.; Topical Delivery of Erythromycin form Various Formulations: An in Vivo Hairless Mouse Study; J. Pharm. Sci.; Vol. 85; N.10; October 1996, 1082 - 84.

## Claims

1. A composition containing solubilized dithranol, said composition comprising a mixture of bilayer vesicles in an oil and water nanoemulsion, wherein an oily lipid phase of said bilayer vesicles and of said nanoemulsion is comprised of a non-ionic oily liquid lipid material that solubilizes dithranol;
**characterised in that**:
said non-ionic oily liquid lipid material is comprised of a-tocopherol acetate alone or in combination with polyoxypropylene-15-stearyl ether,
and **in that**:
said bilayer vesicles are formed *in situ* in a high pressure homogenization step.

2. The composition of claim i, wherein said dithranol is present in the composition in an amount from 0.01% to 1% by weight based upon the total weight of said composition.

3. The composition of claim 1, wherein said oily lipid phase of said vesicles and of said nanoemulsion contains salicylic acid together with dithranol solubilized in said oily lipid phase.

4. The composition of claim 3, wherein salicylic acid is present in the composition in an amount from 0.1% to 3% by weight based upon the weight of the composition.

5. The composition of claim 4, wherein the salicylic acid is present in an amount from 0.5% to 1.5% by weight based upon the weight of the composition.

6. The composition of claim 1, wherein a-tocopherol acetate is present in an amount from 3% to 40% by weight based upon the weight of the composition.

7. The composition of claim 6, wherein a-tocopherol acetate is present in an amount from 5% to 30% by weight based upon the weight of the composition.

8. The composition of claim 7, wherein the oily lipid phase comprises a mixture of polyoxypropylene-15-stearyl ether and a-tocopherol acetate.

9. The composition of claim 1 or claim 2, wherein the composition contains vacuum dried coal tar extract in an amount from 0.1% to about 6% by weight based upon the weight of the composition.

10. The composition of claim 9, wherein the coal tar extract is present an amount from 0.25% to 2.0% by weight of the composition.

11. The composition of claim 1 or claim 2, having incorporated therein a gelling agent in an amount sufficient to form a gel.

12. The composition of claim 1 or claim 2, having incorporated therein a gelling agent in an amount sufficient to form a cream.

13. The composition of claim 1, wherein polyoxypropylene-15-stearyl ether is present in an amount from 1% to 40% by weight based upon the weight of the composition.

14. The composition as claimed in any preceding claim wherein the average particle size of said vesicles is no greater than 450nm.

15. A process for preparing a composition containing solubilized dithranol, said composition comprising a mixture of bilayer vesicles in an oil and water nanoemulsion, wherein an oily lipid phase of said bilayer vesicles and of said nanoemulsion is comprised of a non-ionic oily liquid lipid material that solubilizes dithranol, the process comprising:
a) providing both a solution of dithranol dissolved in a non-ionic oily liquid lipid material and an aqueous medium;
b) mixing said oily solution with said aqueous medium to form a mixture ;
c) homogenizing said mixture to form a homogeneous emulsion containing an oily phase and an aqueous phase, and
d) subjecting said homogeneous mixture to high pressure homogenization at pressures from 138 MPa to 207 MPa (20,000 psi to 30,000 psi);
**characterised in that**:
said non-ionic oily liquid lipid material is comprised of a-tocopherol acetate alone or in combination with polyoxypropylene-15-stearyl ether, and **in that**:
said bilayer vesicles are formed in situ in said high pressure homogenization step (d).

16. The process of claim 15, wherein said dithranol is present in the composition in an amount from 0.1% to 1% by weight based upon the total weight of said composition.

17. The process of claim 15, wherein a-tocopherol acetate is present in an amount from 3% to 40% by weight based upon the weight of the composition.

18. The process of claim 17, wherein the aqueous medium contains natural or semi-synthetic lecithins dissolved therein.

19. The process of claim 18, wherein said oily lipid phase, prior to mixing with said aqueous medium, contains salicylic acid together with dithranol solubilized in said oily lipid phase.

20. The process of claim 19 wherein the salicylic acid is present in an amount from 0.1% to 3% by weight based upon the weight of the composition.

21. The process of claim 20 wherein the salicylic acid is present in an amount from 0.5% to 1.5% by weight upon the weight of the composition.

22. The process of any one of claims 15 to 21 comprising the further step of:
(e) passing the homogeneous mixture obtained from step (d) through a 0.45µm membrane.

## Patentansprüche

1. Zusammensetzung, die solubilisiertes Dithranol enthält, wobei die genannte Zusammensetzung eine Mischung aus Doppelschicht-Vesikeln in einer Öl-und-Wasser-Nanoemulsion umfasst, worin eine ölige Lipidphase der genannten Doppelschicht-Vesikel und der genannten Nanoemulsion aus einem nicht-ionischen, öligen, flüssigen Lipid-Material besteht, das Dithranol solubilisiert;
**dadurch gekennzeichnet, dass**
das genannte nicht-ionische, ölige, flüssige Lipid-Material aus a-Tocopherolacetat alleine oder in Kombination mit Polyoxypropylen-15-stearylether besteht,
und **dadurch**, dass:
die genannten Doppelschicht-Vesikel in situ in einem Hochdruck-Homogenisierungsschritt gebildet werden.

2. Zusammensetzung nach Anspruch 1, worin das genannte Dithranol in der Zusammensetzung in einer Menge von 0,01 Gewichts-% bis 1 Gewichts-%, bezogen auf das Gesamtgewicht der genannten Zusammensetzung, vorliegt.

3. Zusammensetzung nach Anspruch 1, worin die genannte ölige Lipidphase der genannten Vesikel und der genannten Nanoemulsion Salicylsäure gemeinsam mit Dithranol, solubilisiert in der genannten öligen Lipidphase enthält.

4. Zusammensetzung nach Anspruch 3, worin Salicylsäure in der Zusammensetzung in einer Menge von 0,1 Gewichts-% bis 3 % Gewichts-%, bezogen auf das Gewicht der Zusammensetzung, vorliegt.

5. Zusammensetzung nach Anspruch 4, worin die Salicylsäure in einer Menge von 0,5 Gewichts-% bis 1,5 Gewichts-%, bezogen auf das Gewicht der Zusammensetzung, vorliegt.

6. Zusammensetzung nach Anspruch 1, worin a-Tocopherolacetat in einer Menge von 3 Gewichts-% bis 40 Gewichts-%, bezogen auf das Gewicht der Zusammensetzung, vorliegt.

7. Zusammensetzung nach Anspruch 6, worin a-Tocopherolacetat in einer Menge von 5 Gewichts-% bis 30 Gewichts-%, bezogen auf das Gewicht der Zusammensetzung, vorliegt.

8. Zusammensetzung nach Anspruch 7, worin die ölige Lipidphase eine Mischung aus Polyoxypropylen-15-stearylether und a-Tocopherolacetat umfasst.

9. Zusammensetzung nach Anspruch 1 oder Anspruch 2, worin die Zusammensetzung vakuumgetrockneten Steinkohlenteer-Extrakt in einer Menge von 0,1 Gewichts-% bis ungefähr 6 Gewichts-%, bezogen auf das Gewicht der Zusammensetzung, enthält.

10. Zusammensetzung nach Anspruch 9, worin der Steinkohlenteer-Extrakt in einer Menge von 0,25 Gewichts-% bis 2,0 Gewichts-% der Zusammensetzung vorliegt.

11. Zusammensetzung nach Anspruch 1 oder Anspruch 2, in welche ein Geliermittel in einer Menge eingeschlossen ist, die ausreicht, um ein Gel zu bilden.

12. Zusammensetzung nach Anspruch 1 oder Anspruch 2, in welche ein Geliermittel in einer Menge eingeschlossen ist, die ausreicht, um eine Creme zu bilden.

13. Zusammensetzung nach Anspruch 1, worin Polyoxypropylen-15-stearylether in einer Menge von 1 Gewichts-% bis 40 Gewichts-%, bezogen auf das Gewicht der Zusammensetzung, vorliegt.

14. Zusammensetzung, wie in einem der vorstehenden Ansprüche beansprucht, worin die mittlere Partikelgröße der genannten Vesikel nicht größer als 450 nm ist.

15. Verfahren zur Herstellung einer Zusammensetzung, die solubilisiertes Dithranol enthält, wobei die genannte Zusammensetzung eine Mischung aus Doppelschicht-Vesikeln in einer Öl-und-Wasser-Nanoemulsion umfasst, worin eine ölige Lipidphase der genannten Doppelschicht-Vesikel und der genannten Nanoemulsion aus einem nicht-ionischen, öligen, flüssigen Lipid-Material besteht, das Dithranol solubilisiert, wobei das Verfahren umfasst:
a) Bereitstellen von sowohl einer Lösung aus Dithranol, das in einem nicht-ionischen, öligen, flüssigen Lipid-Material gelöst ist, als auch einem wässrigen Medium;
b) Mischen der genannten öligen Lösung mit dem genannten wässrigen Medium, um eine Mischung zu bilden;
c) Homogenisieren der genannten Mischung, um eine homogene Emulsion zu bilden, die eine ölige Phase und eine wässrige Phase enthält, und
d) Unterwerfen der genannten homogenen Mischung einer Hochdruck-Homogenisierung bei Drucken von 138 MPa bis 207 MPa (20.000 psi bis 30.000 psi);
**dadurch gekennzeichnet, dass**:
das genannte nicht-ionische, ölige, flüssige Lipid-Material aus a-Tocopherolacetat alleine oder in Kombination mit Polyoxypropylen-15-stearylether besteht, und **dadurch**, dass:
die genannten Doppelschicht-Vesikel in situ in dem genannten Hochdruck-Homogensierungsschritt (d) gebildet werden.

16. Verfahren nach Anspruch 15, worin das genannte Dithranol in der Zusammensetzung in einer Menge von 0,1 Gewichts-% bis 1 Gewichts-%, bezogen auf das Gesamtgewicht der genannten Zusammensetzung, vorliegt.

17. Verfahren nach Anspruch 15, worin a-Tocopherolacetat in einer Menge von 3 Gewichts-% bis 40 Gewichts-%, bezogen auf das Gewicht der Zusammensetzung, vorliegt.

18. Verfahren nach Anspruch 17, worin das wässrige Medium natürliche oder semi-synthetische Lecithine enthält, die darin gelöst sind.

19. Verfahren nach Anspruch 18, worin die genannte ölige Lipidphase, vor dem Mischen mit dem genannten wässrigen Medium, Salicylsäure gemeinsam mit Dithranol, solubilisiert in der genannten öligen Lipidphase, enthält.

20. Verfahren nach Anspruch 19, worin die Salicylsäure in einer Menge von 0,1 Gewichts-% bis 3 Gewichts-%, bezogen auf das Gewicht der Zusammensetzung, vorliegt.

21. Verfahren nach Anspruch 20, worin die Salicylsäure in einer Menge von 0,5 Gewichts-% bis 1,5 Gewichts-%, bezogen auf das Gewicht der Zusammensetzung, vorliegt.

22. Verfahren nach einem der Ansprüche 15 bis 21, das den weiteren Schritt umfasst:
(e) Durchleiten der aus Schritt (d) erhaltenen homogenen Mischung durch eine 0,45 µm Membran.

## Revendications

1. Composition contenant du dithranol solubilisé, ladite composition comprenant un mélange de vésicules en deux couches dans une nanoémulsion d'huile et d'eau, dans laquelle une phase lipidique huileuse desdites vésicules en deux couches et de ladite nanoémulsion est constituée d'une matière lipidique liquide huileuse non ionique qui solubilise le dithranol ;
**caractérisée en ce que** :
ladite matière lipidique liquide huileuse non ionique est constituée d'acétate d'α-tocophérol seul ou en association avec de l'éther stéarylique de polyoxypropylène-15,
et **en ce que** :
lesdites vésicules en deux couches sont formées in situ dans une étape d'homogénéisation sous haute pression.

2. Composition suivant la revendication 1, dans laquelle ledit dithranol est présent dans la composition en une quantité de 0,01 % à 1 % en poids sur la base du poids total de ladite composition.

3. Composition suivant la revendication 1, dans laquelle ladite phase lipidique huileuse desdites vésicules et de ladite nanoémulsion contient de l'acide salicylique conjointement avec du dithranol solubilisé dans ladite phase lipidique huileuse.

4. Composition suivant la revendication 3, dans laquelle l'acide salicylique est présent dans la composition en une quantité de 0,1 % à 3 % en poids sur la base du poids de la composition.

5. Composition suivant la revendication 4, dans laquelle l'acide salicylique est présent en une quantité de 0,5 % à 1,5 % en poids sur la base du poids de la composition.

6. Composition suivant la revendication 1, dans laquelle l'acétate d'α-tocophérol est présent en une quantité de 3 % à 40 % en poids sur la base du poids de la composition.

7. Composition suivant la revendication 6, dans laquelle l'acétate d'α-tocophérol est présent dans la composition en une quantité de 5 % à 30 % en poids sur la base du poids de la composition.

8. Composition suivant la revendication 7, dans laquelle la phase lipidique huileuse comprend un mélange d'éther stéarylique de polyoxypropylène-15 et d'acétate d'α-tocophérol.

9. Composition suivant la revendication 1 ou la revendication 2, la composition contenant de l'extrait de goudron de houille séché sous vide en une quantité de 0,1 % à environ 6 % en poids sur la base du poids de la composition.

10. Composition suivant la revendication 9, dans laquelle l'extrait de goudron de houille est présent en une quantité de 0,25 % à 2,0 % en poids de la composition.

11. Composition suivant la revendication 1 ou la revendication 2, à laquelle est incorporé un agent gélifiant en une quantité suffisante pour former un gel.

12. Composition suivant la revendication 1 ou la revendication 2, à laquelle est incorporé un agent gélifiant en une quantité suffisante pour former une crème.

13. Composition suivant la revendication 1, dans laquelle l'éther stéarylique de polyoxypropylène-15 est présent en une quantité de 1 % à 40 % en poids sur la base du poids de la composition.

14. Composition suivant l'une quelconque des revendications précédentes, dans laquelle le diamètre moyen de particule desdites vésicules est non supérieur à 450 nm.

15. Procédé pour la préparation d'une composition contenant du dithranol solubilisé, ladite composition comprenant un mélange de vésicules en deux couches dans une nanoémulsion d'huile et d'eau, une phase lipidique huileuse desdites vésicules en deux couches et de ladite nanoémulsion étant constituée d'une matière lipidique huileuse non ionique qui solubilise le dithranol, procédé comprenant les étapes consistant à :
a) fournir une solution de dithranol dissous dans une matière lipidique liquide huileuse non ionique, et un milieu aqueux ;
b) mélanger ladite solution huileuse audit milieu aqueux pour former un mélange ;
c) homogénéiser ledit mélange pour former une émulsion homogène contenant une phase huileuse et une phase aqueuse et
d) soumettre ledit mélange homogène à une homogénéisation sous haute pression à des pressions de 138 MPa à 207 MPa (20 000 psi à 30 000 psi) ;
**caractérisé en ce que** :
ladite matière lipidique liquide huileuse non ionique est constituée d'acétate d'α-tocophérol seul ou en association avec de l'éther stéarylique de polyoxypropylène-15, et **en ce que** :
lesdites vésicules en deux couches sont formées in situ dans ladite étape d'homogénéisation sous pression (d).

16. Procédé suivant la revendication 15, dans lequel ledit dithranol est présent dans la composition en une quantité de 0,1 % à 1 % en poids sur la base du poids total de ladite composition.

17. Procédé suivant la revendication 15, dans lequel l'acétate d'α-tocophérol est présent en une quantité de 3 % à 40 % en poids sur la base du poids de la composition.

18. Procédé suivant la revendication 17, dans lequel le milieu aqueux contient des lécithines naturelles ou semisynthétiques à l'état dissous dans ce milieu.

19. Procédé suivant la revendication 18, dans lequel ladite phase lipidique huileuse, avant d'être mélangée audit milieu aqueux, contient de l'acide salicylique conjointement avec du dithranol solubilisé dans ladite phase lipidique huileuse.

20. Procédé suivant la revendication 19, dans lequel l'acide salicylique est présent en une quantité de 0,1 % à 3 % en poids sur la base du poids de la composition.

21. Procédé suivant la revendication 20, dans lequel l'acide salicylique est présent en une quantité de 0,5 % à 1,5 % en poids sur la base du poids de la composition.

22. Procédé suivant l'une quelconque des revendications 15 à 21, comprenant l'étape supplémentaire :
e) du passage du mélange homogène obtenu dans l'étape (d) à travers une membrane de 0,45 µm.
